(19) Europäisches Patentamt
European Patent Office
Office européen des brevets

(11) **EP 3 492 012 B1**

(12) **EUROPEAN PATENT SPECIFICATION**

(45) Date of publication and mention
of the grant of the patent:
**08.01.2020 Bulletin 2020/02**

(51) Int Cl.:
***A61B 5/11*** *(2006.01)*

(21) Application number: **18020136.0**

(22) Date of filing: **06.04.2018**

(54) **METHOD AND SYSTEM OF OBTAINING HUMAN POSTURE**

VERFAHREN UND SYSTEM ZUR ERFASSUNG EINER MENSCHLICHEN HALTUNG

PROCÉDÉ ET SYSTÈME D'OBTENTION DE POSTURE HUMAINE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **04.12.2017 CN 201711258283**

(43) Date of publication of application:
**05.06.2019 Bulletin 2019/23**

(73) Proprietor: **Chengdu Siwuge Technology Co., Ltd Chengdu (CN)**

(72) Inventor: **Chou, Mi
Chengdu (CN)**

(74) Representative: **Kinnstätter, Klaus
Am Bach 8
96129 Strullendorf (DE)**

(56) References cited:
**US-A- 5 744 953          US-A1- 2005 046 608
US-A1- 2009 278 791      US-A1- 2009 322 763
US-A1- 2017 151 023**

## Description

### CROSS-REFERENCE TO RELATED APPLICATION

The present invention claims priority to pending Chinese Application No. 20171258283X, filed December 4, 2017.

### FIELD OF THE INVENTION

The present invention relates to the technical field of human motion collection, and more particularly to a method and a system of obtaining a human posture.

### BACKGROUND OF THE INVENTION

At present, the main methods of extracting human motion include optical method, acceleration sensor method, time-domain microwaves method and electromagnetic field method. The optical method uses visible light or infrared ray to perform image recognition and tracking on the beacons disposed at human joints, and then realize human motion extraction. The disadvantage is that the beacons can be occluded, resulting in incomplete human motions. Moreover, the image processing speed is slow while the cost is generally high.

The acceleration sensor method obtains the relative position of each of the human joints by calculating the acceleration of human motion. The major disadvantage is that such method can only obtain position information relative to the previous position with a high cost. And the biggest problem is that timing calibration needs to be performed, which is extremely inconvenient to use, causing such method to be only suitable for industrial applications such as film production.

The time-domain microwaves method extracts the positions of the beacons by calculating propagation delays of signals arriving at different receivers. The disadvantage is that the volume of the antenna is so large that the receivers cannot be installed at sufficient positions on the human body. Therefore, such method requires the person to be in a specific space having receivers. The accuracy is low, and the cost of high accuracy positioning (CM level) is also high. Actually, such method is not suitable to capture the human motion and now is mainly used in inventory tracking and logistics management.

The electromagnetic field method contains two kinds of electromagnetic field analysis methods. The first method combines DC magnetic field and alternating electric field to generate a nutation field. Due to the use of DC static field, such method is vulnerable to the interference of the metal in the measurement area. The second method employs a complex transceiver system having three-dimensional orthogonal antenna groups with three frequencies. The three-dimensional orthogonal antenna groups of the transmitting end respectively transmit a signal with one frequency, which the latter is received by the three-dimensional orthogonal antenna groups of the receiving end individually. Then the positions of the receiving antennas on the three coordinate axes can be calculated respectively to further calculate the distance and angle between the receiving antennas and the transmitting antennas. Although such system has a strong anti-interference ability, the structure thereof is complex and the extraction of calculating parameters is complicated. Also, the system is too expensive to be used for common entertainment (at least CNY 100,000 or about 13.000 €). In sum, one common point for those two methods is that the transmitting antennas are disposed at some places independent of a tracked person, while the receiving antennas are disposed on the body of the tracked person as beacons. Therefore, the tracked person must stay within a certain range of the locations of the transmitting antennas, causing the movement of the tracked person to be limited. Document US 2009/0322763 A1 discloses a motion capture apparatus and method.

### BRIEF SUMMARY OF THE INVENTION

In order to solve the aforementioned problems, the present invention provides a method and a system of obtaining a human posture that has the technical effects of convenience, fast response, low cost, and only one calibration.

To achieve the above aims, the present invention provides a method of obtaining a human posture, comprising following steps:

- step 1, transmitting, by one or more first type electromagnetic field radiators installed on a human torso, one or more electromagnetic signals to a processor, and transmitting, by one or more second type electromagnetic field radiators installed on human limbs, one or more electromagnetic signals to the processor;

- step 2, measuring differences of voltage amplitudes of received signals of the first type electromagnetic field radiators relative to an electromagnetic field radiator disposed at origin of a reference coordinate, and calculating coordinate information of the first type electromagnetic field radiators relative to the origin;

- step 3, acquiring motion trajectories of the second type electromagnetic field radiators based on relations of distances and angles between the second type electromagnetic field radiators and first type electromagnetic field radiators, and further acquiring coordinate information of the second type electromagnetic field radiators relative to the origin by calculating coordinate changes based on the motion trajectories; and

- step 4, acquiring real-time information of a human posture based on real-time coordinate information of the first type electromagnetic field radiators relative to the origin and real-time coordinate information of the second type electromagnetic field radiators relative to the origin.

The principle of the present invention is as follows: the first type electromagnetic field radiators and the second electromagnetic field radiators are respectively installed on the human torso and human limbs; each of the electromagnetic field radiators transmits signals to the other electromagnetic field radiators. The electromagnetic field radiators receiving the signals transfer the received signals to the processor; the processor measures differences of the voltage amplitudes of the received signals of the first type electromagnetic field radiators relative to the electromagnetic field radiator disposed at the origin of the reference coordinate, and then calculates coordinate information of the first type electromagnetic field radiators relative to the origin; the processor acquires motion trajectories of the second type electromagnetic field radiators based on relations of distances and angles between the second type electromagnetic field radiators and first type electromagnetic field radiators, and then acquires coordinate information of the second type electromagnetic field radiators relative to the origin by calculating coordinate changes based on the motion trajectories; and the processor obtains real-time information of a human posture based on real-time coordinate information of the first type electromagnetic field radiators relative to the origin and real-time coordinate information of the second type electromagnetic field radiators relative to the origin.

The electromagnetic field radiators are used to transmit and receive signals. For instance, the electromagnetic field radiators disposed at the positions to be measured radiate the signals generated by the transmit waveform generating circuit to the surroundings to form a local field distribution. Then the electromagnetic field radiators disposed at the positions for measuring receive the signals. After being amplified by an amplification circuit, the signals are collected by a data acquisition card and converted from analog signals to digital signals. After digital filtration, the digital signals are transferred into a signal processing circuit to perform a computing process.

Generally, the origin of the coordinate is a certain point on the tracked person's back which can be set according to actual needs. The change of the human posture is able to be obtained based on the coordinate changes of the first type electromagnetic field radiators relative to the origin and the coordinate changes of the second type electromagnetic field radiators relative to the origin.

Moreover, the electromagnetic field radiators and the processor are installed directly or indirectly on a human body; and connections between the processor and the electromagnetic field radiators are wired or wireless.

Furthermore, in step 1, after measuring the differences of the voltage amplitudes of the received signals of the first type electromagnetic field radiators relative to the electromagnetic field radiator disposed at the origin of the reference coordinate, calculating the coordinate information of the first type electromagnetic field radiators relative to the origin based on corresponding relation between the voltage amplitudes and coordinate.

In accordance with the invention, the relations of distances and angles between the second type electromagnetic field radiators and first type electromagnetic field radiators in step 3 are obtained in view of a following step: acquiring a distance relation of the second type electromagnetic field radiators and the first type electromagnetic field radiators, and acquiring an angle relation of the second type electromagnetic field radiators and the first type electromagnetic field radiators, wherein one of the second type electromagnetic field radiators 2H transmits signals that are received by two of the first type electromagnetic field radiators 2G and 2F; signal strengths of the signals received by 2G and 2F are measured to obtain Vout2g and Vout2f; a plurality of values of $r$ and $\theta$ are calculated based on Vout2g and Vout2f wherein $r$ refers to a distance between 2H and 2G, and $\theta$ refers to an angle between 2H and 2G; and the plurality of values of $r$ and $\theta$ are constrained according to preset constraints of $r$ and $\theta$ to eventually obtain the distance relation and the angle relation of 2H and 2G.

In addition, the constraints of $r$ and $\theta$ are determined by the movement range of human joints corresponding to installation positions of electromagnetic field radiators. The movement range varies from different human joints, and can be obtained by the exercise physiological analysis which will not be described in detail in the present invention.

Furthermore, the present invention correspondingly provides a system of obtaining a human posture, comprising: a plurality of first type electromagnetic field radiators installed on a human torso, a plurality of second type electromagnetic field radiators installed on human limbs, and a processor, wherein all of the first type electromagnetic field radiators and the second type electromagnetic field radiators transmit a plurality of electromagnetic signals to the processor; the processor measures differences of the voltage amplitudes of the received signals of the first type electromagnetic field radiators relative to the electromagnetic field radiator disposed at the origin of the reference coordinate, and then calculates coordinate information of the first type electromagnetic field radiators relative to the origin; the processor acquires motion trajectories of the second type electromagnetic field radiators based on relations of distances and angles between the second type electromagnetic field radiators and first type electromagnetic field radiators, and then acquires coordinate information of the second type electromagnetic field radiators relative to the origin by calculating coordinate changes based on the motion trajectories; and the processor obtains real-time information of the human posture based on real-time coordinate information of the first type electromagnetic field radiators relative to the origin and real-time coordinate information of the second type electromagnetic field radiators relative to the origin.

Preferably, the electromagnetic field radiators and the processor are installed directly or indirectly on a human body; and connections between the processor and the electromagnetic field radiators are wired or wireless.

Furthermore, the processor measures the differences of the voltage amplitudes of the received signals of the first type electromagnetic field radiators relative to the electromagnetic field radiator disposed at the origin of the reference coordinate, and then calculates the coordinate information of the first type electromagnetic field radiators relative to the origin based on corresponding relation between the voltage amplitudes and coordinate.

Moreover, the relations of distances and angles between the second type electromagnetic field radiators and first type electromagnetic field radiators are obtained by the processor, more particularly, a distance relation of the second type electromagnetic field radiators and the first type electromagnetic field radiators and an angle relation of the second type electromagnetic field radiators and the first type electromagnetic field radiators are obtained. Specifically, one of the second type electromagnetic field radiators 2H transmits signals that are received by two of the first type electromagnetic field radiators 2G and 2F; signal strengths of the signals received by 2G and 2F are measured to obtain Vout2g and Vout2f; a plurality of values of r and $\theta$ are calculated based on Vout2g and Vout2f wherein r refers to a distance between 2H and 2G, and $\theta$ refers to an angle between 2H and 2G; and the plurality of values of r and $\theta$ are constrained according to preset constraints of r and $\theta$ to eventually obtain the distance relation and the angle relation of 2H and 2G.

Preferably, the constraints of r and $\theta$ are determined by the movement range of human joints corresponding to installation positions of electromagnetic field radiators.

The one or more technical schemes provided in the present invention have at least the following technical effects or benefits:

- as all of the miniature electromagnetic field radiators are disposed on the tracked person's body and no external device is needed, the present invention realizes tracking independent of the environment so as to obviously improve conveniences;
- moreover, the present invention achieves tracking absolute coordinate of the position and speed information, avoiding error accumulation, which the latter avoids the complex process for timing calibration;
- furthermore, compared with the optical method and mechanics structures such as an accelerometer and a compass, the measurement of electromagnetic field adopted by the present invention avoids the optical image processing time and the mechanical response time, resulting in fast processing speed;

meanwhile, the whole system has technical effects of simple configuration, low-cost hardware and only one calibration.

For making the above and other purposes, features and benefits become more readily apparent to those ordinarily skilled in the art, the preferred embodiments and the detailed description with accompanying drawings will be put forward in the following description.

BRIEF DESCRIPTION OF THE DRAWINGS

The present invention will become more readily apparent to those ordinarily skilled in the art after reviewing the following detailed description and accompanying drawings, in which:

FIG. 1    is a schematic flowchart of a method of obtaining a human posture provided in the present invention;

FIG. 2    is a schematic diagram of a hardware composition of a system of obtaining a human posture provided in the present invention; and

FIG. 3    is a schematic diagram of a structure of each of the electromagnetic field radiators provided in the present invention.

DETAILED DESCRIPTION OF THE PREFERRED EMBODIMENTS

The present invention will now be described more specifically with reference to the following embodiments. It is to be noted that the following descriptions of preferred embodiments of this invention are presented herein for purpose of illustration and description only. It is not intended to be exhaustive or to be limited to the precise form disclosed.

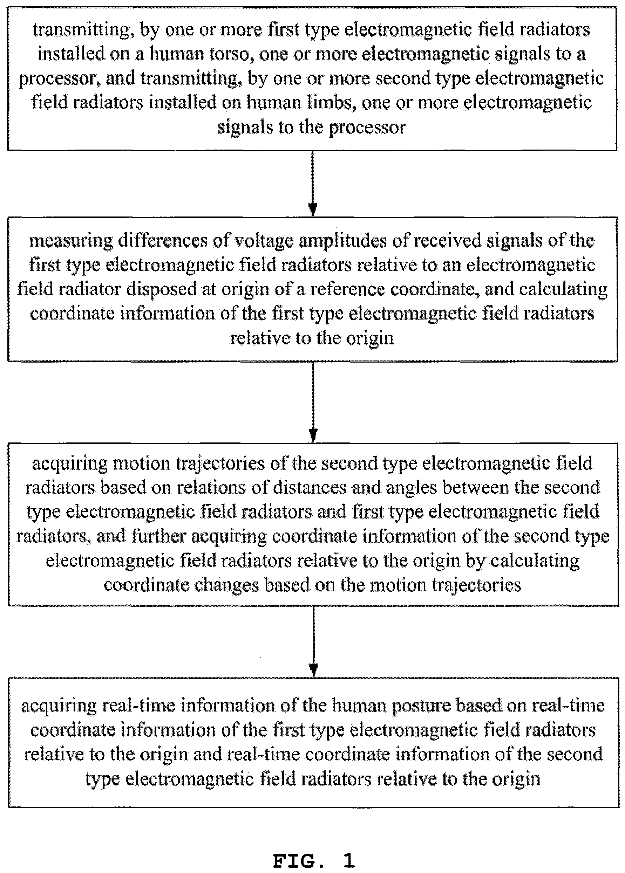

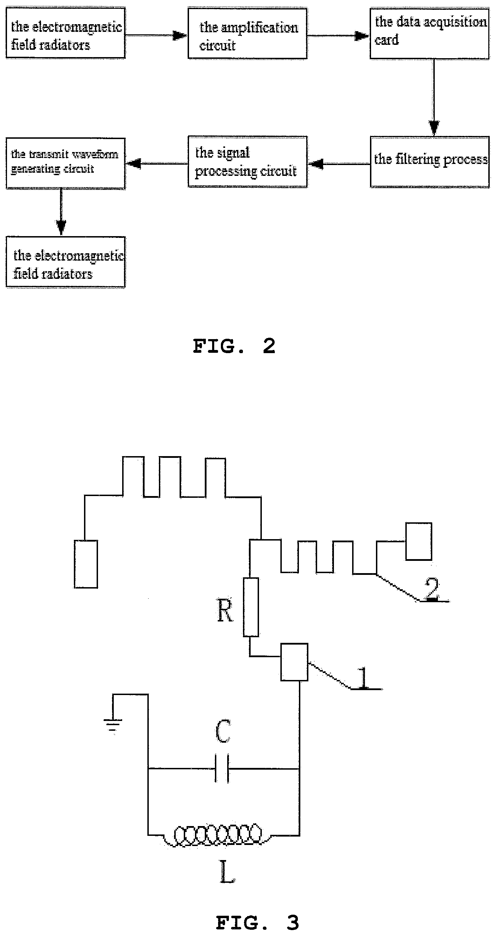

As shown in FIG. 1 and FIG. 2, the present invention provides a method of obtaining a human posture. The method comprises following steps:

a plurality of first type electromagnetic field radiators installed on a human torso, a plurality of second type electromagnetic field radiators installed on human limbs, and a processor; all of the first type electromagnetic field radiators and the second type electromagnetic field radiators transmit a plurality of electromagnetic signals to the processor; the processor measures differences of voltage amplitudes of received signals of the first type electromagnetic field radiators relative to an electromagnetic field radiator disposed at origin of a reference coordinate, and then calculates coordinate information of the first type electromagnetic field radiators relative to the origin; the processor acquires motion trajectories of the second type electromagnetic field radiators based on relations of distances and angles between the second type electromagnetic field radiators and first type electromagnetic field radiators, and then acquires coordinate information of the second type electromagnetic field radiators relative to the origin by calculating coordinate changes based on the motion trajectories; and the processor obtains real-time information of a human posture based on real-time coordinate information of the first type electromagnetic field radiators relative to the origin and real-time coordinate information of the second type electromagnetic field radiators relative to the origin.

The electromagnetic field radiators provided in the present invention include first type electromagnetic field radiators and second type electromagnetic field radiators, wherein each of the electromagnetic field radiators comprises an access port for signal access, a capacitance connected between the access port and ground, a coil connected in parallel with the capacitance, and an oscillator connected to the access port. The access port is connected to a cable, allowing the signals to feed from the cable. Thus the electromagnetic energy can feed to the coil and the oscillator via the capacitance. Low frequency signals mainly radiate from the coil to form a near field distribution, while high frequency signals mainly radiate from the oscillator to form a near field distribution. The electromagnetic field radiators convert the guided electromagnetic waves in the cable into a near quasi-stationary electromagnetic field distribution, and enable the low frequency signals and the high frequency signals to feed simultaneously, realizing two frequency spectrum radiation components with large frequency difference. Meanwhile, the coil and the oscillator can convert the surrounding quasi-stationary electromagnetic field into the induced current, which flows to the cable to form the receiving signal. The aforementioned circuit configuration is not only simple and small, but also provides an enough radiation area to obtain a sufficient radiation distance which is up to 1 meter, filling the technical blanks of the wireless charging coil and the NFC antenna.

As shown in FIG. 3, each of the electromagnetic field radiators comprises the access port 1, the capacitance C, the coil L and the oscillator 2, wherein the access port 1 is connected to the cable for signal access; the capacitance C is connected between the access port 1 and ground; the coil L is connected in parallel with the capacitance C; and the oscillator 2 is connected to the access port 1. When the signals are accessed through the cable, the low frequency signals and the high frequency signals in the electromagnetic waves respectively radiate from the coil and the oscillator to form the near field distribution. Each of the electromagnetic field radiators can further comprises a port for realizing the connection between the capacitance and the ground. For example, the port is connected to a grounded housing.

The hardware composition of the system is shown in FIG. 2. The electromagnetic field radiators are disposed throughout the body of the tracked person and connected to the processor via the cable. The electromagnetic field radiators are used to transmit and receive signals. For instance, the electromagnetic field radiators disposed at the positions to be measured radiate the signals generated by the transmit waveform generating circuit to the surroundings to form a local field distribution. Then the electromagnetic field radiators disposed at the positions for measuring receive the signals. After being amplified by an amplification circuit, the signals are collected by a data acquisition card and converted from analog signals to digital signals. After digital filtration, the digital signals are transferred into a signal processing circuit to perform an arithmetic processing calculation.

The amplification circuit uses the hybrid integrated circuit of Infineon Corporation established by simulating the BJT triode. The data acquisition card uses the A/D chip with low cost and high-precision developed by TI Corporation. The filtering process is performed by employing the digital filter software established by processor resource, and processed by software. The signal processing circuit employs an ARM7 or ARM9 series chip, or a FPGA series chip with low cost developed by Altera Corporation. The transmit waveform is generated by the processor together with the peripheral operational amplifier and the crystal oscillator circuit.

The electromagnetic field radiators are disposed on the tracked person's garment or the belt binding device. The processor is connected with each of the electromagnetic field radiators through the cable to calculate the amplitudes and the phases of the signals transmitted or received by each of the electromagnetic field radiators, and further calculate the distance and angle between any two of the electromagnetic field radiators. After all distances and angles between any two of the electromagnetic field radiators are determined, the action posture of the human body can be also determined.

The algorithm for calculating the relative positions of the electromagnetic field radiators is as follows: most of the electromagnetic field radiators disposed on the back or the chest of the human torso are the first type electromagnetic field radiators. The first type electromagnetic field radiators use the central area of the back as the origin of the reference coordinate. When the body is in a natural upright state, the initial position of each of the first type electromagnetic field radiators is known. In the process of human movement, the relative positions of the first type electromagnetic field radiators change in a one-dimensional direction due to the limited movement range of the human torso. Consequently, with the measurement of the differences of the voltage amplitudes of the received signals of the first type electromagnetic field radiators relative to one of the first type electromagnetic field radiators that is disposed at the origin of the reference coordinate, the change amount of the positions of the measured first type electromagnetic field radiators relative to the origin can be calculated directly.

The remaining electromagnetic field radiators disposed on the human torso and the electromagnetic field radiators disposed on the human limbs are the second type electromagnetic field radiators. The motion trajectories of the second type electromagnetic field radiators are determined according to the relations of distances and angles between the second type electromagnetic field radiators and first type electromagnetic field radiators. Subsequently, the coordinate information of the second type electromagnetic field radiators relative to the origin can be acquired by calculating coordinate changes.

The calculating method for the relative positions of the second type electromagnetic field radiators and the first type electromagnetic field radiators is as follows: According to the calculation formula for the electromagnetic field induced voltage:

$$dB = k\frac{Id1 \cdot a_R}{R^2}$$

In the aforementioned calculation formula, dB refers to a magnetic flux density segment in Tesla, wherein one Tesla equals one Weber per square meter (Wb/m$^2$); dl refers to a current segment in a current direction; $a_R$ refers to a unit vector from dl to point P; R refers to a distance from the current segment dl to point P; and K is the proportionality constant.

According to the Biot-Savart law of the space magnetic field formula, the magnetic flux density of any current segment formed at a certain point in space is inversely proportional to square of the distance between the certain point and the current segment, and proportional to the cross product of the included angle between the certain point and the direction of the current segment.

When a signal is transmitted by one of the electromagnetic field radiators (i.e. transmitting electromagnetic field radiator) and received by another one of the electromagnetic field radiators (i.e. receiving electromagnetic field radiator), according to the integral form of Faraday's law in Max-well equation, with the same frequency, the signal strength (i.e. voltage amplitude) received by the receiving electromagnetic field radiator is determined by the magnetic flux density of coil of the receiving electromagnetic field radiator generated by the transmitting electromagnetic field radiator. The signal strength is linear with the magnetic flux density. On the other hand, the shape of the antenna and the turns of the coil are constant so that their contributions to the total magnetic flux density of the coil obtained by integrating according to the Biot-Savart law are also constant. Therefore, the magnetic flux density is only determined by the distance and the angle between the receiving electromagnetic field radiator and the transmitting electromagnetic field radiator.

For example, the serial number of one of the second electromagnetic field radiators disposed on the thigh is 2H. When being tracked, 2H transmits signals received by two of the first electromagnetic field radiators, 2G and 2F.

The movement range of 2H relative to 2G and 2F is limited by the human joints and muscle actions. In relation to 2G, 2H is only able to move within a distance of 4-8 cm (depending on raising the thigh and lateral valgus motion) and an angle of 0-130°. Similarly, in relation to 2F, 2H is only able to move within a distance of 6-10 cm (depending on raising the thigh and lateral valgus motion) and an angle of 0-130°.

Thus, within the movement range described above, when 2G and 2F receive the signals transmitted from 2H, the measured signal strength Vout2g and Vout2f also correspond to the distance r relation and the included angle θ relation of 2H and 2G. The relations are as follows:

```
Vout2g = f(r1,θ1);

    4 < r1 < 8;

    0 < θ1 < 130;

Vout2f = f(r2,θ2)

    6 < r2 < 10;

    0 < θ2 < 130;
```

wherein r1 refers to a distance between 2G and 2H; θ1 refers to an angle between 2G and 2H; r2 refers to a distance between 2F and 2H; and θ2 refers to an angle between 2F and 2H. The calculation formula for f(r,θ) is as follows:

$$V = \frac{A \cdot \mu \cdot N_1 \cdot N_2}{4\pi}\oint_{c_1}\oint_{c_2}\frac{\cos\theta dl_1 dl_2}{r} ;$$

In the above formula, A refers to the voltage transfer coefficient that is a constant related to the circuit configuration of each of the electromagnetic field radiators; μ refers to the air permeability; N2 refers to the turns of the coil of the transmitting electromagnetic field radiator; N1 refers to the turns of the coil of the receiving electromagnetic field radiator; C1 refer to the integration along the transmitting coil loop; C2 refer to the integration along the receiving coil loop; θ refers to the included angle between the transmitting coil and the receiving coil; r refers to the distance between the transmitting current segment and the receiving current segment; and $dl_1$ and $dl_2$ are integration units.

In view of the above formula, the voltage amplitudes of the receiving electromagnetic field radiator disposed at the known position and angle can be calculated. With the measured voltage amplitudes of the receiving electromagnetic field radiator, the measured values of Vout2g and Vout2f, the constraints of r and θ, and the position/angle-voltage matrix calculated according to the aforementioned formula, the position/angle point of the matrix that has a lowest root mean square error with the test voltage is calculated by Newton optimization method of minimum criterion of root mean square error (various similar maturation algorithms for solving such problem are known in the prior art, such as Runge-Kutta method). Finally, the corresponding parameters of r1, θ1, r2 and θ2 of 2H are calculated. After coordinate conversion, the real-time coordinate of 2H relative to the origin is acquired.

The amount of the receiving electromagnetic radiators for tracking one of the electromagnetic field radiators can be increased when the computational efficiency is poor.

The positioning method for the other electromagnetic field radiators is similar to the method described above. During the calculation of the other electromagnetic field radiators, the constraints of r and θ are determined by the movement range of human joints where the electromagnetic field radiators locate. Such movement range can be obtained by the exercise physiological analysis. The global absolute coordinate of the mark points of the human motion (i.e. the positions of the electromagnetic field radiators) relative to a certain fixed point (i.e. the origin) disposed on the back can be obtained by the processor with the determination of relative positional relationships of all the electromagnetic field radiators. When in use, the tracked person starts with a standard motion which is used by the system to complete the calibration process. Later, the tracked person can move freely without additional calibration processes.

While the invention has been described in terms of what is presently considered to be the most practical and preferred embodiments, it is to be understood that the invention needs not be limited to the disclosed embodiment. On the contrary, it is intended to cover various modifications and similar arrangements included within the scope of the appended claims which are to be accorded with the broadest interpretation so as to encompass all such modifications and similar structures.

## Claims

1. A method of obtaining a human posture, comprising following steps:

   step 1, transmitting, by one or more first type electromagnetic field radiators installed on a human torso, one or more electromagnetic signals received from another electromagnetic field radiator to a processor, and transmitting, by one or more second type electromagnetic field radiators installed on human limbs, one or more electromagnetic signals received from another electromagnetic field radiator to the processor;

   step 2, measuring, by the processor, differences of voltage amplitudes of received signals of the first type electromagnetic field radiators relative to an electromagnetic field radiator disposed at origin of a reference coordinate, and calculating coordinate information of the first type electromagnetic field radiators relative to the origin;

   step 3, acquiring, by the processor, motion trajectories of the second type electromagnetic field radiators based on relations of distances and angles between the second type electromagnetic field radiators and first type electromagnetic field radiators, and further acquiring coordinate information of the second type electromagnetic field radiators relative to the origin by calculating coordinate changes based on the motion trajectories; and

   step 4, obtaining, by the processor, real-time information of the human posture based on real-time coordinate information of the first type electromagnetic field radiators relative to the origin and real-time coordinate information of the second type electromagnetic field radiators relative to the origin,

   wherein the relations of distances and angles between the second type electromagnetic field radiators and first type electromagnetic field radiators in step 3 are obtained according to a following step:

   acquiring a distance relation of the second type electromagnetic field radiators and the first type electromagnetic field radiators, and acquiring an angle relation of the second type electromagnetic field radiators and the first type electromagnetic field radiators,

   wherein one of the second type electromagnetic field radiators 2H transmits signals that are received by two of the first type electromagnetic field radiators 2G and 2F; signal strengths of the signals received by 2G and 2F are measured to obtain Vout2g and Vout2f; a plurality of values of r and θ are calculated based on Vout2g and Vout2f wherein r refers to a distance between 2H and 2G, and θ refers to an angle between 2H and 2G; and the plurality of values of r and θ are constrained according to preset constraints of r and θ to eventually obtain the distance relation and the angle relation of 2H and 2G.

2. The method of obtaining the human posture according to claim 1, wherein electromagnetic field radiators and the processor are installed directly or indirectly on a human body; and connections between the processor and the electromagnetic field radiators are wired or wireless.

**3.** The method of obtaining the human posture according to claim 1 or 2, in step 1, after measuring the differences of the voltage amplitudes of the received signals of the first type electromagnetic field radiators relative to the electromagnetic field radiator disposed at the origin of the reference coordinate, calculating the coordinate information of the first type electromagnetic field radiators relative to the origin based on corresponding relation between the voltage amplitudes and coordinate.

**4.** The method of obtaining the human posture according to claim 1, 2 or 3, wherein the constraints of r and θ are determined by the movement range of human joints corresponding to installation positions of electromagnetic field radiators.

**5.** The method of obtaining the human posture according to one of claims 1 to 4, wherein the electromagnetic field radiators are used to transmit and receive signals; the electromagnetic field radiators disposed at positions to be measured radiate signals generated by a transmit waveform generating circuit to surroundings to form a local field distribution; the electromagnetic field radiators disposed at positions for measuring receive the signals; the signals are amplified by an amplification circuit; the signals are collected by a data acquisition card and converted from analog signals to digital signals; and after digital filtration, the digital signals are transferred into a signal processing circuit to perform an computing process.

**6.** A system of obtaining a human posture, comprising:

a plurality of first type electromagnetic field radiators installed on a human torso, a plurality of second type electromagnetic field radiators installed on human limbs, and a processor; wherein all of the first type electromagnetic field radiators and the second type electromagnetic field radiators transmit a plurality of electromagnetic signals received from other electromagnetic field radiators to the processor;

the processor measures differences of voltage amplitudes of received signals of the first type electromagnetic field radiators relative to an electromagnetic field radiator disposed at origin of a reference coordinate, and then calculates coordinate information of the first type electromagnetic field radiators relative to the origin;

the processor acquires motion trajectories of the second type electromagnetic field radiators based on relations of distances and angles between the second type electromagnetic field radiators and first type electromagnetic field radiators, and then acquires coordinate information of the second type electromagnetic field radiators relative to the origin by calculating coordinate changes based on the motion trajectories; and

the processor obtains real-time information of the human posture based on real-time coordinate information of the first type electromagnetic field radiators relative to the origin and real-time coordinate information of the second type electromagnetic field radiators relative to the origin, wherein the relations of distances and angles between the second type electromagnetic field radiators and first type electromagnetic field radiators are obtained by the processor, wherein a distance relation of the second type electromagnetic field radiators and the first type electromagnetic field radiators and an angle relation of the second type electromagnetic field radiators and the first type electromagnetic field radiators are obtained; one of the second type electromagnetic field radiators 2H transmits signals that are received by two of the first type electromagnetic field radiators 2G and 2F; signal strengths of the signals received by 2G and 2F are measured to obtain Vout2g and Vout2f; a plurality of values of r and θ are calculated based on Vout2g and Vout2f wherein r refers to a distance between 2H and 2G, and θ refers to an angle between 2H and 2G; and the plurality of values of r and θ are constrained according to preset constraints of r and θ to eventually obtain the distance relation and the angle relation of 2H and 2G.

**7.** The system of obtaining the human posture according to claim 6, wherein electromagnetic field radiators and the processor are installed directly or indirectly on a human body; and connections between the processor and the electromagnetic field radiators are wired or wireless.

**8.** The system of obtaining the human posture according to claim 6 or 7, wherein the processor measures the differences of the voltage amplitudes of the received signals of the first type electromagnetic field radiators relative to the electromagnetic field radiator disposed at the origin of the reference coordinate, and then calculates the coordinate information of the first type electromagnetic field radiators relative to the origin based on corresponding relation between the voltage amplitudes and coordinate.

**9.** The system of obtaining the human posture according to claim 6, 7 or 8, wherein the constraints of r and θ are determined by the movement range of human joints corresponding to installation positions of electromagnetic field radiators.

**10.** The system of obtaining the human posture according to one of claims 6 to 9, further comprising: an amplification circuit, a data acquisition card, a processing circuit and a transmit waveform generating circuit, wherein electromagnetic field radiators are used to transmit and receive signals; the electromagnetic field radiators disposed at positions to be measured radiate signals generated by the transmit waveform generating circuit to surroundings to form a local field distribution; the electromagnetic field radiators disposed at positions for measuring receive the signals; the signals are amplified by the amplification circuit; the signals are collected by the data acquisition card and converted from analog signals to digital signals; the digital signals are digital filtered by the processing circuit; and then the digital signals are transmitted into the processor to perform an computing process.

**Patentansprüche**

1. Verfahren zur Erlangung der Haltung eines Menschen, umfassend folgende Schritte:

Schritt 1, Übermitteln, mittels eines oder mehrerer elektromagnetischer Feldstrahler eines ersten Typs, die an einem menschlichen Rumpf angeordnet sind, eines oder mehrerer elektromagnetischer Signale, die von anderen elektromagnetischen Feldstrahlern empfangen werden, an einen Prozessor und
Übermitteln, mittels eines oder mehrerer elektromagnetischer Feldstrahler eines zweiten Typs, die an menschlichen Gliedern angeordnet sind, eines oder mehrerer elektromagnetischer Signale, die von anderen elektromagnetischen Feldstrahlern empfangen werden an den Prozessor;
Schritt 2, Messen, mittels des Prozessors, Differenzen von Spannungsamplituden empfangener Signale der elektromagnetischen Feldstrahler des ersten Typs relativ zu einem elektromagnetischen Feldstrahler, der an einem Ursprung einer Referenzkoordinate angeordnet ist, und Berechnen von Koordinateninformationen der elektromagnetischen Feldstrahler des ersten Typs relativ zu dem Ursprung;
Schritt 3, Erlangen, mittels des Prozessors, Bewegungstrajektorien der elektromagnetischen Feldstrahler des zweiten Typs basierend auf Beziehungen von Entfernungen und Winkeln zwischen den elektromagnetischen Feldstrahlern des zweiten Typs und den elektromagnetischen Feldstrahlern des ersten Typs, und weiterhin Erlangen von Koordinateninformationen der elektromagnetischen Feldstrahler des zweiten Typs relativ zum Ursprung durch Berechnen von Koordinatenänderungen basierend auf den Bewegungstrajektorien; und

Schritt 4, Erlangen, mittels des Prozessors, von Echtzeitinformationen über die Haltung des Menschen basierend auf Echtzeit-Koordinateninformation der elektromagnetischen Feldstrahler des ersten Typs relativ zum Ursprung und Echtzeit-Koordinateninformation der elektromagnetischen Feldstrahler des zweiten Typs relativ zum Ursprung,

wobei die Beziehungen der Entfernungen und Winkeln zwischen den elektromagnetischen Feldstrahlern des zweiten Typs und den elektromagnetischen Feldstrahlern des ersten Typs in Schritt 3 entsprechend einem folgenden Schritt erlangt werden:

Erlangen einer Abstandsbeziehung der elektromagnetischen Feldstrahler des zweiten Typs und der elektromagnetischen Feldstrahler des ersten Typs und Erlangen einer Winkelbeziehung der elektromagnetischen Feldstrahler des zweiten Typs und der elektromagnetischen Feldstrahler des ersten Typs,
wobei einer der der elektromagnetischen Feldstrahler des zweiten Typs 2H Signale übermittelt, die von zwei elektromagnetischen Feldstrahlern des ersten Typs 2G und 2F empfangen werden; Signalstärken der Signale, die von 2G und 2F empfangen werden, gemessen werden, um Vout2G und Vout2f zu erlangen; basierend auf Vout2G und Vout2f eine Vielzahl von Werten für r und $\theta$ berechnet wird, wobei r sich auf einen Abstand zwischen 2H und 2 GG bezieht und 0 sich auf einen Winkel zwischen 2H und 2 GG bezieht; und die Vielzahl von Werten für r und $\theta$ entsprechend vorgegebenen Randbedingungen für r und $\theta$ beschränkt wird, um schließlich die Abstandsbeziehung und die Winkelbeziehung von 2H und 2G zu erlangen.

2. Verfahren zur Erlangung der Haltung eines Menschen nach Anspruch 1, wobei elektromagnetische Feldstrahler und der Prozessor direkt oder indirekt am Körper eines Menschen installiert sind; und Verbindungen zwischen dem Prozessor und den elektromagnetischen Feldstrahlern verdrahtet oder drahtlos sind.

3. Verfahren zur Erlangung der Haltung eines Menschen nach Anspruch 1 oder 2, in Schritt 1, nach dem Messen der Differenzen der Spannungsamplituden der empfangenen Signale der Feldstrahler des ersten Typs relativ zu dem Feldstrahler am Ursprung der Referenzkoordinate, Berechnung der Koordinateninformation der Feldstrahler des ersten Typs relativ zum Ursprung basierend auf einer Korrespondenzbeziehung zwischen den Spannungsamplituden und den Koordinaten.

4. Verfahren zur Erlangung der Haltung eines Menschen nach Anspruch 1, 2 oder 3, wobei die Randbedingungen für r und θ durch den Bewegungsbereich menschlicher Gelenke bezüglich der Anordnungspositionen der elektromagnetischen Feldstrahler bestimmt sind.

5. Verfahren zur Erlangung der Haltung eines Menschen nach einem der Ansprüche 1 bis 4, wobei die elektromagnetischen Feldstrahler dazu verwendet werden, um Signale auszusenden und zu empfangen; wobei die elektromagnetischen Feldstrahler, die an zu messenden Positionen angeordnet sind, in die Umgebung Signale ausstrahlen, die von einem Sendewellenformgenerierungsschaltkreis generiert werden, um eine lokale Feldverteilung zu formen; wobei die elektromagnetischen Feldstrahler, die an Positionen zum Messen angeordnet sind, die Signale empfangen; wobei die Signale durch einen Verstärkungskreis verstärkt werden; wobei die Signale von einer Datenerfassungskarte gesammelt werden und von analogen Signalen zu digitalen Signalen konvertiert werden; und wobei die digitalen Signale nach einer digitalen Filterung in einen Signalverarbeitungskreis übertragen werden, um einen Rechenprozess durchzuführen.

6. System zur Erlangung der Haltung eines Menschen, umfassend:

eine Mehrzahl von elektromagnetischen Feldstrahlern eines ersten Typs, die an einem menschlichen Rumpf angeordnet sind, eine Mehrzahl von elektromagnetischen Feldstrahlern eines zweiten Typs, die an menschlichen Gliedern angeordnet sind, und einen Prozessor; wobei alle elektromagnetischen Feldstrahler des ersten Typs und des zweiten Typs eine Mehrzahl von elektromagnetischen Signalen, die sie von anderen elektromagnetischen Feldstrahlern empfangen haben, an den Prozessor übermitteln; wobei der Prozessor Differenzen von Spannungsamplituden empfangener Signale der elektromagnetischen Feldstrahler des ersten Typs relativ zu einem elektromagnetischen Feldstrahler, der an einem Ursprung einer Referenzkoordinate angeordnet ist, misst und dann Koordinateninformationen der elektromagnetischen Feldstrahler des ersten Typs relativ zu dem Ursprung berechnet; wobei der Prozessor Bewegungstrajektorien der elektromagnetischen Feldstrahler des zweiten Typs basierend auf Beziehungen von Entfernungen und Winkeln zwischen den elektromagnetischen Feldstrahlern des zweiten Typs und den elektromagnetischen Feldstrahlern des ersten Typs erlangt und dann Koordinateninformationen der elektromagnetischen Feldstrahler des zweiten Typs relativ zum Ursprung durch Berechnen von Koordinatenänderungen basierend auf den Bewegungstrajektorien erlangt; wobei der Prozessor Echtzeitinformationen über die Haltung des Menschen basierend auf Echtzeit-Koordinateninformation der elektromagnetischen Feldstrahler des ersten Typs relativ zum Ursprung und Echtzeit-Koordinateninformation der elektromagnetischen Feldstrahler des zweiten Typs relativ zum Ursprung erlangt; wobei die Beziehungen der Entfernungen und Winkeln zwischen den elektromagnetischen Feldstrahlern des zweiten Typs und den elektromagnetischen Feldstrahlern des ersten Typs vom Prozessor erlangt werden, wobei eine Abstandsbeziehung der elektromagnetischen Feldstrahler des zweiten Typs und der elektromagnetischen Feldstrahler des ersten Typs und eine Winkelbeziehung der elektromagnetischen Feldstrahler des zweiten Typs und der elektromagnetischen Feldstrahler des ersten Typs erlangt werden, wobei einer der der elektromagnetischen Feldstrahler des zweiten Typs 2H Signale übermittelt, die von zwei elektromagnetischen Feldstrahlern des ersten Typs 2G und 2F empfangen werden; Signalstärken der Signale, die von 2G und 2F empfangen werden, gemessen werden, um Vout2G und Vout2f zu erlangen; basierend auf Vout2G und Vout2f eine Vielzahl von Werten für r und θ berechnet wird, wobei r sich auf einen Abstand zwischen 2H und 2 GG bezieht und θ sich auf einen Winkel zwischen 2H und 2 GG bezieht; und die Vielzahl von Werten für r und θ entsprechend vorgegebenen Randbedingungen für r und θ beschränkt wird, um schließlich die Abstandsbeziehung und die Winkelbeziehung von 2H und 2G zu erlangen.

7. System zur Erlangung der Haltung eines Menschen nach Anspruch 6, wobei elektromagnetische Feldstrahler und der Prozessor direkt oder indirekt am Körper eines Menschen installiert sind; und Verbindungen zwischen dem Prozessor und den elektromagnetischen Feldstrahlern verdrahtet oder drahtlos sind.

8. System zur Erlangung der Haltung eines Menschen nach Anspruch 6 oder 7, wobei der Prozessor die Differenzen der Spannungsamplituden der empfangenen Signale der Feldstrahler des ersten Typs relativ zu dem Feldstrahler am Ursprung der Referenzkoordinate misst und dann die Koordinateninformation der Feldstrahler des ersten Typs relativ zum Ursprung basierend auf einer Korrespondenzbeziehung zwischen den Spannungsamplituden und den Koordinaten berechnet.

**9.** System zur Erlangung der Haltung eines Menschen nach Anspruch 6, 7 oder 8, wobei die Randbedingungen für r und θ durch den Bewegungsbereich menschlicher Gelenke bezüglich der Anordnungspositionen der elektromagnetischen Feldstrahler bestimmt sind.

**10.** System zur Erlangung der Haltung eines Menschen nach einem der Ansprüche 6 bis 9, weiterhin umfassend: einen Verstärkungskreis, eine Datenerfassungskarte, einen Signalverarbeitungskreis und einen Sendewellenformgenerierungsschaltkreis, wobei die elektromagnetischen Feldstrahler dazu verwendet werden, um Signale auszusenden und zu empfangen; die elektromagnetischen Feldstrahler, die an zu messenden Positionen angeordnet sind, in die Umgebung Signale ausstrahlen, die von dem Sendewellenformgenerierungsschaltkreis generiert werden, um eine lokale Feldverteilung zu formen; wobei die elektromagnetischen Feldstrahler, die an Positionen zum Messen angeordnet sind, die Signale empfangen; wobei die Signale von dem Verstärkungskreis verstärkt werden; wobei die Signale von der Datenerfassungskarte gesammelt werden und von analogen Signalen zu digitalen Signalen konvertiert werden; wobei digitale Signale von dem Signalverarbeitungskreis Digital gefiltert werden; und wobei dann die digitalen Signale in den Prozessor übertragen werden, um einen Rechenprozess durchzuführen.

## Revendications

**1.** Procédé d'obtention d'une posture humaine, comprenant les étapes suivantes :

étape 1, la transmission, par un ou plusieurs émetteurs de champ électromagnétique de premier type installés sur un torse humain, d'un ou de plusieurs signaux électromagnétiques reçus en provenance d'un autre émetteur de champ électromagnétique à un processeur, et la transmission, par un ou plusieurs émetteurs de champ électromagnétique de second type installés sur des membres humains, d'un ou de plusieurs signaux électromagnétiques reçus en provenance d'un autre émetteur de champ électromagnétique au processeur ;
étape 2, la mesure, par le processeur, de différences d'amplitudes de tension de signaux reçus des émetteurs de champ électromagnétique de premier type par rapport à un émetteur de champ électromagnétique disposé à l'origine d'une coordonnée de référence, et le calcul d'informations de coordonnées des émetteurs de champ électromagnétique de premier type par rapport à l'origine ;

étape 3, l'acquisition, par le processeur, de trajectoires de mouvement des émetteurs de champ électromagnétique de second type sur la base de relations de distances et d'angles entre les émetteurs de champ électromagnétique de second type et les émetteurs de champ électromagnétique de premier type, et l'acquisition en outre d'informations de coordonnées des émetteurs de champ électromagnétique de second type par rapport à l'origine en calculant des changements de coordonnées sur la base des trajectoires de mouvement ; et
étape 4, l'obtention, par le processeur, d'informations en temps réel de la posture humaine sur la base d'informations de coordonnées en temps réel des émetteurs de champ électromagnétique de premier type par rapport à l'origine et des informations de coordonnées en temps réel des émetteurs de champ électromagnétique de second type par rapport à l'origine,

dans lequel les relations de distances et d'angles entre les émetteurs de champ électromagnétique de second type et les émetteurs de champ électromagnétique de premier type à l'étape 3 sont obtenues selon une étape suivante :

l'acquisition d'une relation de distance des émetteurs de champ électromagnétique de second type et des émetteurs de champ électromagnétique de premier type, et l'acquisition d'une relation d'angle des émetteurs de champ électromagnétique de second type et des émetteurs de champ électromagnétique de premier type,
dans lequel l'un des émetteurs de champ électromagnétique de second type 2H transmet des signaux qui sont reçus par deux des émetteurs de champ électromagnétique de premier type 2G et 2F ; des intensités de signal des signaux reçus par 2G et 2F sont mesurées pour obtenir Vout2g et Vout2f ; une pluralité de valeurs de r et θ sont calculées sur la base de Vout2g et Vout2f, dans lequel r désigne une distance entre 2H et 2G, et θ désigne un angle entre 2H et 2G ; et la pluralité de valeurs de r et θ sont contraintes selon des contraintes préétablies de r et θ pour obtenir finalement la relation de distance et la relation d'angle de 2H et 2G.

**2.** Procédé d'obtention de la posture humaine selon la revendication 1, dans lequel des émetteurs de champ électromagnétique et le processeur sont installés directement ou indirectement sur un corps humain ; et des connexions entre le processeur et les émetteurs de champ électromagnétique sont filaires ou sans fil.

**3.** Procédé d'obtention de la posture humaine selon la revendication 1 ou 2, comprenant à l'étape 1, après la mesure des différences des amplitudes de tension des signaux reçus des émetteurs de champ électromagnétique de premier type par rapport à l'émetteur de champ électromagnétique disposé à l'origine de la coordonnée de référence, le calcul des informations de coordonnées des émetteurs de champ électromagnétique de premier type par rapport à l'origine sur la base d'une relation correspondante entre les amplitudes de tension et la coordonnée.

**4.** Procédé d'obtention de la posture humaine selon l'une des revendications 1, 2 ou 3, dans lequel les contraintes de r et θ sont déterminées par la plage de déplacement d'articulations humaines correspondant à des positions d'installation d'émetteurs de champ électromagnétique.

**5.** Procédé d'obtention de la posture humaine selon l'une des revendications 1 à 4, dans lequel les émetteurs de champ électromagnétique sont utilisés pour transmettre et recevoir des signaux ; les émetteurs de champ électromagnétique disposés à des positions à mesurer émettent des signaux générés par un circuit de génération de forme d'onde de transmission autour d'eux pour former une distribution de champ local ; les émetteurs de champ électromagnétique disposés à des positions de mesure reçoivent les signaux ; les signaux sont amplifiés par un circuit d'amplification ; les signaux sont collectés par une carte d'acquisition de données et convertis de signaux analogiques en signaux numériques ; et après un filtrage numérique, les signaux numériques sont transférés dans un circuit de traitement de signal pour réaliser un processus informatique.

**6.** Système d'obtention d'une posture humaine, comprenant :

une pluralité d'émetteurs de champ électromagnétique de premier type installés sur un torse humain, une pluralité d'émetteurs de champ électromagnétique de second type installés sur des membres humains, et un processeur ; dans lequel tous les émetteurs de champ électromagnétique de premier type et les émetteurs de champ électromagnétique de second type transmettent une pluralité de signaux électromagnétiques reçus en provenance d'autres émetteurs de champ électromagnétique au processeur ;
le processeur mesure des différences d'amplitudes de tension de signaux reçus des émetteurs de champ électromagnétique de premier type par rapport à un émetteur de champ électromagnétique disposé à l'origine d'une coordonnée de référence, puis calcule des informations de coordonnées des émetteurs de champ électromagnétique de premier type par rapport à l'origine ;
le processeur acquiert des trajectoires de mouvement des émetteurs de champ électromagnétique de second type sur la base de relations de distances et d'angles entre les émetteurs de champ électromagnétique de second type et les émetteurs de champ électromagnétique de premier type, puis acquiert des informations de coordonnées des émetteurs de champ électromagnétique de second type par rapport à l'origine en calculant des changements de coordonnées sur la base des trajectoires de mouvement ; et
le processeur obtient des informations en temps réel de la posture humaine sur la base d'informations de coordonnées en temps réel des émetteurs de champ électromagnétique de premier type par rapport à l'origine et des informations de coordonnées en temps réel des émetteurs de champ électromagnétique de second type par rapport à l'origine,
dans lequel les relations de distances et d'angles entre les émetteurs de champ électromagnétique de second type et les émetteurs de champ électromagnétique de premier type sont obtenues par le processeur, dans lequel une relation de distance des émetteurs de champ électromagnétique de second type et des émetteurs de champ électromagnétique de premier type, et une relation d'angle des émetteurs de champ électromagnétique de second type et des émetteurs de champ électromagnétique de premier type sont obtenues ; l'un des émetteurs de champ électromagnétique de second type 2H transmet des signaux qui sont reçus par deux des émetteurs de champ électromagnétique de premier type 2G et 2F ; des intensités de signal des signaux reçus par 2G et 2F sont mesurées pour obtenir Vout2g et Vout2f ; une pluralité de valeurs de r et θ sont calculées sur la base de Vout2g et Vout2f, dans lequel r désigne une distance entre 2H et 2G, et θ désigne un angle entre 2H et 2G ; et la pluralité de valeurs de r et θ sont contraintes selon des contraintes préétablies de r et θ pour obtenir finalement la relation de distance et la relation d'angle de 2H et 2G.

**7.** Système d'obtention d'une posture humaine selon la revendication 6, dans lequel des émetteurs de champ électromagnétique et le processeur sont installés directement ou indirectement sur un corps humain ; et des connexions entre le processeur et les émetteurs de champ électromagnétique sont filaires ou sans fil.

**8.** Système d'obtention d'une posture humaine selon la revendication 6 ou 7, comprenant à l'étape 1, après la mesure des différences des amplitudes de tension des signaux reçus des émetteurs de champ électromagnétique de premier type par rapport à l'émetteur de champ électromagnétique disposé à l'origine de la coordonnée de référence, le calcul des informations de coordonnées des émetteurs de champ électromagnétique de premier type par rapport à l'origine sur la base d'une relation correspondante entre les amplitudes de tension et la coordonnée.

9. Système d'obtention d'une posture humaine selon l'une des revendications 6, 7 ou 8, dans lequel les contraintes de r et θ sont déterminées par la plage de déplacement d'articulations humaines correspondant à des positions d'installation d'émetteurs de champ électromagnétique.

10. Système d'obtention d'une posture humaine selon l'une des revendications 6 à 9, dans lequel les émetteurs de champ électromagnétique sont utilisés pour transmettre et recevoir des signaux ; les émetteurs de champ électromagnétique disposés à des positions à mesurer émettent des signaux générés par un circuit de génération de forme d'onde de transmission autour d'eux pour former une distribution de champ local ; les émetteurs de champ électromagnétique disposés à des positions de mesure reçoivent les signaux ; les signaux sont amplifiés par un circuit d'amplification ; les signaux sont collectés par une carte d'acquisition de données et convertis de signaux analogiques en signaux numériques ; et après un filtrage numérique, les signaux numériques sont transférés dans un circuit de traitement de signal pour réaliser un processus informatique.

transmitting, by one or more first type electromagnetic field radiators installed on a human torso, one or more electromagnetic signals to a processor, and transmitting, by one or more second type electromagnetic field radiators installed on human limbs, one or more electromagnetic signals to the processor

measuring differences of voltage amplitudes of received signals of the first type electromagnetic field radiators relative to an electromagnetic field radiator disposed at origin of a reference coordinate, and calculating coordinate information of the first type electromagnetic field radiators relative to the origin

acquiring motion trajectories of the second type electromagnetic field radiators based on relations of distances and angles between the second type electromagnetic field radiators and first type electromagnetic field radiators, and further acquiring coordinate information of the second type electromagnetic field radiators relative to the origin by calculating coordinate changes based on the motion trajectories

acquiring real-time information of the human posture based on real-time coordinate information of the first type electromagnetic field radiators relative to the origin and real-time coordinate information of the second type electromagnetic field radiators relative to the origin

FIG. 1

FIG. 2

FIG. 3

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 201711258283X

- US 20090322763 A1